# EUROPÄISCHE PATENTSCHRIFT

(11) **EP 2 581 096 B1**
(45) Veröffentlichungstag und Bekanntmachung des Hinweises auf die Patenterteilung: **16.12.2015**
(21) Anmeldenummer: 12184121.7
(22) Anmeldetag: 12.09.2012
(51) Int. Cl.: A61L 2/08, B65B 55/02, B67C 7/00, H01J 33/02, H01J 37/30

(54) **Vorrichtung und Verfahren zum Sterilisieren von Behältnissen mit in die Behältnisse eingeführter Elektronenquelle**
Device and method for sterilising containers with electron source introduced into the containers
Dispositif et procédé de stérilisation de récipients avec source des éléctrons introduite dans les récipients

(30) Priorität: 30.09.2011 DE 102011054097
(43) Veröffentlichungstag der Anmeldung: 17.04.2013
(73) Patentinhaber: Krones AG, 93073 Neutraubling (DE)
(72) Erfinder: Knott, Josef, 93073 Neutraubling (DE); Engelhard, Patrick, 93073 Neutraubling (DE); Scheuren, Hans, 93073 Neutraubling (DE)
(74) Vertreter: Bittner, Bernhard

(56) Entgegenhaltungen:
- EP-A1- 2 161 202
- WO-A1-97/07024
- WO-A2-2009/095182
- US-A1- 2011 012 032
- US-B2- 7 520 108

## Beschreibung

Die vorliegende Erfindung bezieht sich auf ein Verfahren und eine Vorrichtung zum Sterilisieren von Behältnissen und insbesondere von Kunststoffbehältnissen. Die Sterilisation eines zu befüllenden Behälters ist neben dem eigentlichen Füllvorgang ein zentraler Prozessschritt insbesondere in einer aseptischen Abfüllanlage. Die möglichen Sterilisationsformen variieren hinsichtlich der Entkeimungsmittel und der Prozessführung. Allen gemein ist jedoch, dass die abtötende Wirkung aufgrund chemischer Prozesse bewirkt wird. Neuere aus dem Stand der Technik bekannte Entwicklungen grenzen sich hiervon ab und nutzen ionisierende Strahlung, um eine Keimreduzierung zu erreichen. Diese Strahlung besteht in den meisten Anwendungen aus beschleunigten Ladungsträgern und insbesondere beschleunigten Elektronen, die in einer entsprechenden Anlage erzeugt und in den zu sterilisierenden Behälter eingeführt werden. Der Vorteil dieser Vorgehensweise besteht in der Verringerung oder Vermeidung des Einsatzes chemischer Stoffe. Allerdings weisen diese Vorrichtungen jedoch teilweise noch nicht etablierte und dadurch teure Beschleunigungsanlagen auf und weiterhin eine vergleichsweise komplizierte Verschaltung und Überwachung. Daneben muss durch die entstehende Störstrahlung eine Abschirmung um die Gesamtanlage oder um einzelne Teile dieser Anlage vorhanden sein.

US 7,520,108 B2 offenbart eine Desinfektionsvorrichtung zur keimtötenden Behandlung von Behältern umfassend eine in die Behältnisse einführbaren Elektronenquelle.

Aus der DE 10 2009 008633 A1 ist eine Vorrichtung zum Sterilisieren von Behältnissen bekannt. Dabei werden die Behältnisse entlang eines vorgegebenen Transportpfades transportiert, genauer durch einen Behandlungsraum. Eine Sterilisation erfolgt hier durch Beaufschlagung der Behältnisse mit Elektronen, wobei eine derartige Sterilisationseinrichtung eine Erzeugungseinrichtung zum Erzeugen der Elektronen aufweist, sowie eine Beschleunigungseinrichtung, um die von der Erzeugungseinrichtung erzeugten Elektronen in Richtung der in dem Behandlungsraum befindlichen Behältnisse zu beschleunigen. Dabei liegt ein Gasdruck innerhalb des Behandlungsraumes unter einem Gasdruck außerhalb des Behandlungsraumes. Es wird daher vorgeschlagen, dass die Elektronen in einem Bereich beschleunigt werden, der unter einem relativ geringen Druck steht, um so die Reichweite dieser Elektronen zu erhöhen.

Die aktuellen am Markt erhältlichen Systeme, welche zur Sterilisation eingesetzt werden, weisen eine Elektronenerzeugungsvorrichtung, sowie eine Bündelung auf, sowie mechanische Lösungen zur Übersetzung des Elektronenstrahles in das zu sterilisierende Behältnis. Genauer gesagt wird bei diesen Anlagen üblicherweise ein Strahlfinger in die Behältnisse eingeführt und die Elektronen werden durch diesen Strahlfinger hindurch in Richtung eines Austrittsfensters beschleunigt und treten anschließend aus dem Austrittsfenster aus und auf die Innenwand der Behältnisse. Um hier eine Wirkung und Übertragung des Strahles zu gewährleisten, müssen die Elektronen zumeist stark beschleunigt werden. Die dazu benötigte Beschleunigungsspannung beträgt bis zu 150 kV. Die kinetische Energie der Elektronen wird entsprechend erhöht und es entsteht im selben Maße unerwünschte radioaktive bzw. Röntgenstrahlung.

Ein weiterer Nachteil bisheriger Anlagen und Vorrichtungen zur Erzeugung von Elektronenstrahlen ist die Problematik des Transportes der Elektronen von der Erzeugerquelle hin zum Wirkungsort. Die im Zwischenraum vorhandenen Moleküle, wie sie in der Luft oder in dem vorhandenen Austrittsfenster auftreten, behindern in entscheidendem Maße die Reichweite der Elektronenstrahlen. Als Folge davon muss die Beschleunigung der Elektronen angepasst werden, wobei dies ein Schritt ist, der wiederum die Größe, die Komplexität, das Gefährdungspotential und die Abschirmung, sowie die Kosten der Gesamtanlage in die Höhe treibt.

Der vorliegenden Erfindung liegt daher die Aufgabe zu Grunde, ein Verfahren und eine Vorrichtung zur Verfügung zu stellen, welche den Einsatz sehr hoher Beschleunigungsspannungen vermeiden. Auch sollen insgesamt die Kosten für eine derartige Anlage zum Sterilisieren von Behältnissen reduziert werden. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Im Folgenden bedentet "Ladungsträger" "Elektronen". Erfindungsgemäß wird die Ladungsträgererzeugung wenigstens teilweise in das Behältnis eingeführt und erzeugt im Inneren des Behältnisses die Ladungsträger.

Es wird vorgeschlagen, dass die Elektronenerzeugung an sich in das zu sterilisierende Behältnis gelegt wird. Damit werden nunmehr die Ladungsträger erst innerhalb des Behältnisses erzeugt. Auf diese Weise kann auf das Vorsehen sehr hoher Beschleunigungsspannungen zum Beschleunigen der Elektronen verzichtet werden. Zwischen der Elektronenerzeugungseinrichtung und der Innenwand des Behältnisses sind üblicherweise nur sehr geringe Distanzen zurückzulegen, so dass keine hohe Beschleunigung der Elektronen erforderlich ist.

Bei einem weiteren bevorzugten Verfahren wird die Ladungsträgererzeugungseinrichtung in einer Längsrichtung des Behältnisses in dieses eingeführt. Vorteilhaft werden die Behältnisse während der Sterilisation selbst bewegt - insbesondere in eine Richtung, welche sich von der Einführungsrichtung der Ladungsträgererzeugungseinrichtung in die Behältnisse unterscheidet. Wie oben erwähnt, handelt es sich bei den Ladungsträgern insbesondere um Elektronen.

Bei einem weiteren vorteilhaften Verfahren wird innerhalb des Behältnisses wenigstens zeitweise vor oder während des Sterilisationsvorganges ein Unterdruck (gegenüber einem atmosphärischen Druck) erzeugt. So ist es beispielsweise möglich, dass innerhalb des Behältnisses eine Elektronenwolke in einem Grob- oder Feinvakuum erzeugt wird, welches sich wiederum in dem zu sterilisierenden Behältnis befindet. Auf diese Weise ist es möglich, dass die Elektronen beinahe spontan zu der entsprechenden Wirkoberfläche gefördert werden. Die hierfür benötigte Energie kann aufgrund fehlender Störmoleküle annähernd und vorteilhaft auch ohne weitere Beschleunigungsspannung aufgebracht werden. Die Elektronen deaktivieren an der zu sterilisierenden Oberfläche durch Ionisation Zellkompartimente und Moleküle der ungewünschten Mikroorganismen.

Bei einem weiteren vorteilhaften Verfahren wird im Inneren des Behältnisses ein Druck erzeugt, der zwischen 0,0005 mbar und 800 mbar, bevorzugt zwischen 0,0005 mbar und 600 mbar, bevorzugt zwischen 0,001 mbar und 300 mbar und besonders bevorzugt zwischen 0,001 mbar und 100 mbar liegt. Bei diesen Druckverhältnissen haben die Elektronen eine relativ hohe Reichweite, so dass, wie erwähnt, auf zusätzliche Beschleunigungsspannungen jedenfalls jedoch auf sehr hohe Beschleunigungsspannungen verzichtet werden kann. Die Beschleunigungsspannungen, falls verwendet, liegen vorteilhaft unter 100 kV, bevorzugt unter 50 kV, bevorzugt unter 10 kV.

Vorteilhaft handelt es sich bei dem zu sterilisierenden Behältnis um einen Kunststoffvorformling. Derartige Kunststoffvorformlinge sind aus mehreren Gründen besonders für die hier vorgenommene Sterilisation geeignet. Zum einen weisen diese üblicherweise einen zylindrischen Grundkörper auf, so dass der Abstand zwischen der Elektronenerzeugungseinrichtung und der Innenwandung des Kunststoffvorformlings entlang der Längsrichtung des Kunststoffvorformlings im Wesentlichen konstant bleibt. Daneben weisen die Kunststoffvorformlinge auch eine sehr hohe Stabilität auf und können daher mit einem Unterdruck beaufschlagt werden. Bei anderen Behältnissen könnte ein derartiger Unterdruck auch zu einem Verformen oder Implodieren des Behältnisses führen.

Es wäre auch möglich, dass die Behältnisse während des Sterilisationsvorganges bzw. während der Aktivierung der Ladungsträgererzeugungseinrichtung bezüglich dieser gedreht werden, insbesondere um eine Längsachse. Auf diese Weise kann eine gleichmäßige Bestrahlung der Innenwandung der Behältnisse auch dann sichergestellt werden, wenn die Elektronen in Umfangsrichtung nicht gleichmäßig ausgegeben werden. Es wäre dann auch möglich, die Elektronen ausgehend von der Erzeugungseinrichtung nun in einer Vorzugsrichtung zu beschleunigen. Bevorzugt werden hier die Behältnisse gedreht, es wäre jedoch auch möglich, die innerhalb der Behältnisse befindliche Ladungsträgererzeugungseinrichtung um die bevorzugte Längsachse zu drehen.

Bei einer weiteren vorteilhaften Ausführungsform werden die Behältnisse während ihrer Sterilisation entlang eines vorgegebenen Transportpfades transportiert. Vorteilhaft handelt es sich dabei um einen kreisförmigen Transportpfad.

Bei einer weiteren vorteilhaften Ausführungsform werden die Kunststoffvorformlinge durch einen Unterdruck an einer Halteeinrichtung gehalten. Es wird daher vorgeschlagen, dass die Kunststoffvorformlinge an eine Halteeinrichtung übergeben werden, dann mit einem Unterdruck beaufschlagt werden und mit diesem Unterdruck auch an dieser Halteeinrichtung gehalten werden.

Es wird in diesem Zusammenhang darauf hingewiesen, dass ein derartiger Unterdruck gegebenenfalls auch in Glasbehältnisse erzeugt werden kann, so dass auch diese möglicherweise für die hier beschriebene Sterilisation geeignet sind.

Die vorliegende Erfindung ist weiterhin auf eine Vorrichtung zum Sterilisieren von Behältnissen und insbesondere von Kunststoffbehältnissen gemäß Anspruch 6 gerichtet.

Es wird also auch vorrichtungsseitig vorgeschlagen, dass die Ladungsträgererzeugungseinrichtung selbst in das Behältnis eingeführt wird (und nicht etwa ein Austrittsfenster, über welches stark beschleunigte Elektronen austreten können. Vorteilhaft ist die Ladungsträgererzeugungseinrichtung durch eine Mündung des Behältnisses in dieses einführbar. Im Gegensatz zum Stand der Technik wird also vorteilhaft kein Austrittsfenster in die Behältnisse eingeführt, sondern die Erzeugungseinrichtung selbst.

Bei einer weiteren vorteilhaften Ausführungsform weist die Vorrichtung eine Transporteinrichtung auf, welche die Behältnisse wenigstens zeitweise während deren Sterilisation transportiert. Daneben weist die Vorrichtung auch eine Bewegungseinrichtung auf, welche die Beaufschlagungseinrichtung in das Behältnis einführt. Dabei ist es möglich, dass die Ladungsträgererzeugungseinrichtung in einer Längsrichtung des Behältnisses bewegt wird, vorteilhaft bleibt jedoch die Ladungsträgererzeugungseinrichtung in dieser Längsrichtung fest und das Behältnis wird dieser gegenüber bewegt.

Bei einer vorteilhaften Ausführungsform weist die Vorrichtung einen drehbaren Träger auf, an dem eine Vielzahl von Sterilisationseinrichtungen angeordnet ist. Bei einer weiteren vorteilhaften Ausführungsform weist die Vorrichtung eine Druckbeaufschlagungseinrichtung auf, welche die Behältnisse mit einem Unterdruck beaufschlagt. Insbesondere auch durch die Verwendung dieses Unterdrucks kann die Reichweite der Elektronen im Inneren der Behältnisse erhöht werden. Es wäre jedoch in manchen Fällen auch denkbar, dass ohne die Erzeugung eines Unterdrucks bereits eine gewisse Sterilisation möglich ist. Weiterhin wäre es auch möglich, nicht nur die Behältnisse selbst mit dem Unterdruck zu beaufschlagen, sondern den Raum, in dem die Behältnisse gefördert werden. Vorteilhaft werden jedoch lediglich die Innenräume der Behältnisse mit dem besagten Unterdruck beaufschlagt. Bei einer weiteren vorteilhaften Ausführungsform weist die Vorrichtung eine Ablenkeinrichtung auf, welche die Ladungsträger in Richtung der Innenwand der Behältnisse richtet. So könnte beispielsweise eine positive Elektrode außerhalb der Kunststoffbehältnisse und diese Kunststoffbehältnisse umgebend vorgesehen sein.

So könnte beispielsweise eine Glühkathode in das Innere der Behältnisse eingeführt werden. Dabei wäre es auch möglich, diese Glühkatode ohne einen diese umgebenden Glaskolben einzuführen und das nötige Vakuum in dem Behältnis selbst zu erzeugen. Bei einer vorteilhaften Ausführungsform weist die Strahlungserzeugungseinrichtung einen ersten in das Behältnis einführbaren, stromdruchfließbaren, elektrischen Leiter auf. Durch den besagten Stromfluss werden die Elektronen, wie an sich aus dem Stand der Technik bekannt, freigesetzt. Dabei kann es sich beispielsweise um eine Wolframkathode handeln.

Bei einer weiteren vorteilhaften Ausführungsform kann die Strahlungserzeugungseinrichtung einen zweiten in das Behältnis einführbaren, stromdruchfließbaren, elektrischen Leiter aufweisen. Dieser zweite Leiter kann dabei vorteilhaft parallel zu dem ersten Leiter geschaltet sein. Dabei kann die Ausgestaltung so sein, dass bei einem Ausfall des oben erwähnten ersten stromdurchflossenen Leiters der zweite Leiter aktiviert wird, so dass auch bei Ausfall eines elektrischen Leiters der Sterilisationsprozess fortgeführt werden kann.

Die vorliegende Erfindung ist weiterhin auf eine Anlage zum Behandeln von Kunststoffbehältnissen gerichtet. Erfindungsgemäß weist diese Anlage eine Vorrichtung der oben bezeichneten Art auf. Vorteilhaft weist die Anlage auch eine Vorrichtung zum Umformen von Kunststoffvorformlingen zu Kunststoffbehältnissen auf. Bei einer weiteren vorteilhaften Ausführungsform ist die oben erwähnte Vorrichtung in der Transportrichtung der Kunststoffbehältnisse vor der Vorrichtung zum Umformen von Kunststoffvorformlingen zu Kunststoffbehältnissen angeordnet. Bei einer weiteren vorteilhaften Ausführungsform weist die Anlage auch eine Erwärmungseinrichtung zum Erwärmen von Kunststoffvorformlingen auf und diese Erwärmungseinrichtung ist dabei in der Transportrichtung der Kunststoffvorformlinge vor der Vorrichtung zum Umformen von Kunststoffvorformlingen zu Kunststoffbehältnissen angeordnet.

Die erfindungsgemäße Vorrichtung kann dabei in der Transportrichtung der Kunststoffvorformlinge zwischen der Erwärmungseinrichtung und der Vorrichtung zum Umformen von Kunststoffvorformlingen zu Kunststoffbehältnissen angeordnet sein. Vorteilhaft ist jedoch die erfindungsgemäße Vorrichtung vor der Erwärmungseinrichtung zum Erwärmen von Kunststoffvorformlingen angeordnet, beispielsweise vor oder in einem Eingangsbereich dieser Erwärmungsvorrichtung.

Weitere Vorteile und Ausführungsformen ergeben sich aus den beigefügten Zeichnungen: Darin zeigen:
- Fig. 1a, 1b: zwei schematische Darstellungen zur Veranschaulichung des der Erfindung zugrunde liegenden Problems;
- Fig. 2: eine Seitenansicht einer erfindungsgemäßen Vorrichtung;
- Fig.3: eine Detailansicht einer erfindungsgemäßen Vorrichtung und
- Fig.4: eine Detailansicht einer erfindungsgemäßen Vorrichtung in einer weiteren Ausführungsform.

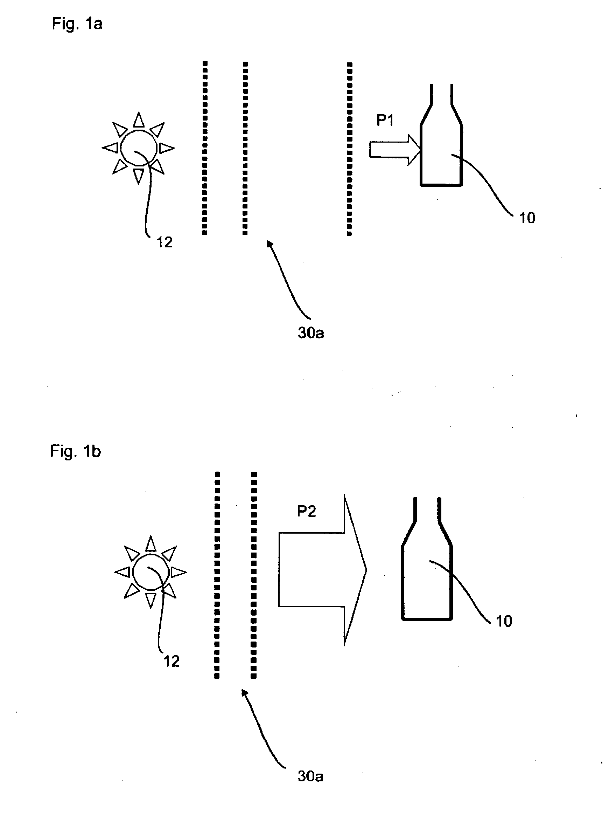

Die Figuren 1 a und 1 b zeigen zwei Darstellungen zur Veranschaulichung des der Erfindung zugrunde liegenden Problems. Dabei ist jeweils eine Ladungsträgererzeugungseinrichtung dargestellt 12, welche Ladungsträger erzeugt. Zwischen dieser Ladungsträgererzeugungseinrichtung 12 und dem Behältnis 10 befinden sich mehrere Barrieren 30a, die hier durch punktierte Linien dargestellt sind. So kann beispielsweise ein Austrittsfenster einer Sterilisationseinrichtung, Luft, usw. eine derartige Barriere darstellen. Damit ist einer der Hauptnachteile bisheriger Anlagen und Vorrichtungen zur Erzeugung von Elektronenstrahlen die Problematik des Transports der Elektronen von der Erzeugerquelle hin zum Wirkungsort. Die im Zwischenraum vorhandenen Moleküle, wie sie in der Luft oder auch in dem vorhandenen Austrittsfenster auftreten, behindern in entscheidendem Maße die Reichweite der Elektronenstrahlen.

Als Folge davon muss die Beschleunigung der Elektronen angepasst werden, ein Schritt, der wiederum die Größe, die Komplexität, das Gefährdungspotenzial, die Abschirmung und die Kosten der Gesamtanlage in die Höhe treibt.

Um diese Verschlechterungen für eine weitere Elektronenreichweite nicht durchführen zu müssen und dennoch eine ausreichende Sterilisation zu bewirken, wird vorgeschlagen, Maßnahmen zu ergreifen, so dass die benötigten Elektronen mit geringerem Abstand von der Ladungsträgererzeugungsquelle hin zu dem Wirkungsort transportiert werden. Dies ist in Figur 1 b veranschaulicht. In diesem Falle werden die Barrieren 30a verringert, so dass im Ergebnis die Elektronen eine höhere Laufstrecke aufweisen. Der im Vergleich zu dem Pfeil P1 größere Pfeil P2 veranschaulicht hier schematisch die größere Anzahl an Elektronen, die zu dem Behältnis 10 bzw. genauer gesagt auf dessen Innenwandung gelangen.

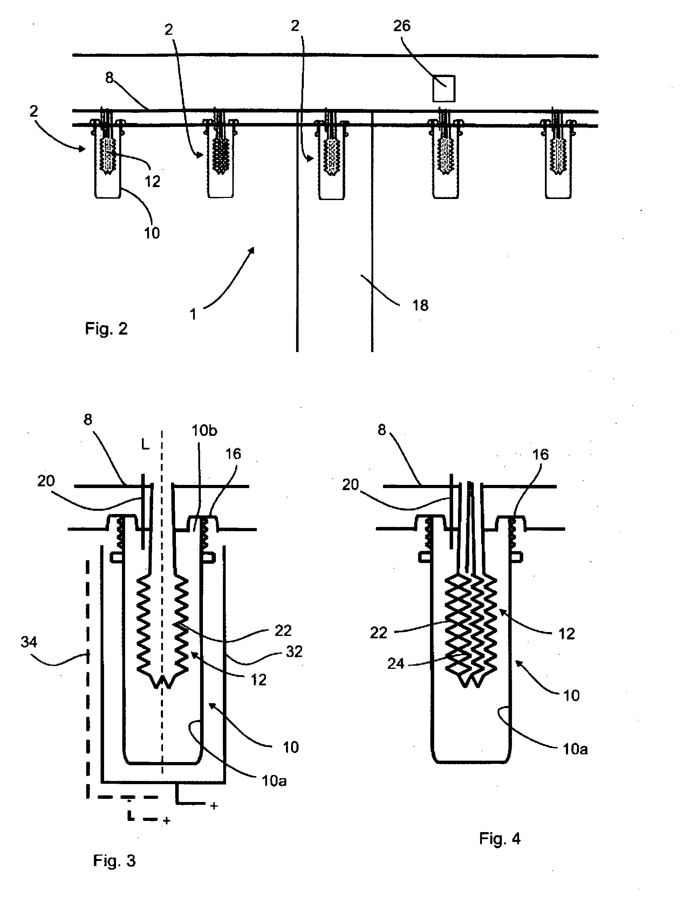

Fig. 2 zeigt eine schematische Darstellung einer erfindungsgemäßen Vorrichtung 1. In diesem Fall ist ein um eine Achse bzw. mittels einer Welle 18 drehbarer Träger 8 vorgesehen, an dem eine Vielzahl von Behandlungsstationen bzw. Sterilisationseinrichtungen 2 vorhanden ist.

Der Vorrichtung 1 können dabei die jeweiligen Behältnisse 10 mittels einer Greifeinrichtung übergeben werden, wobei diese Greifeinrichtung die Kunststoffbehältnisse vorteilhaft von außen her und insbesondere unterhalb der Tragringe greift. Anschließend werden die Behältnisse mittels einer Halterung fixiert. Genauer gesagt kann an jeder Station bzw. Sterilisationseinrichtung 2 über einen Außengreifer eines zuführenden Sternes das Behältnis bzw. der Kunststoffvorformling in einem entsprechend ausgeformten Andockbereich gelenkt werden. Das Bezugszeichen 26 kennzeichnet schematisch eine Hubeinrichtung, welche beispielsweise einen Kunststoffvorformling an den Träger 8 derart heranfährt, dass die Elektronenerzeugungseinrichtung in das Innere des Kunststoffbehältnisses gelangt. Damit wird vorteilhaft das Kunststoffbehältnis in die Mitte gefahren oder gestülpt. Die Halsoberfläche des Kunststoffvorformlings berührt dabei einen Andockbereich. Zu Beginn einer Unterdruckeinstellung kann der Außengreifer des Übergabesternes das Kunststoffbehältnis noch halten und bei einem ausreichenden Unterdruck (beispielsweise 800 mbar oder kleiner) an dem Außengreifer des Zufuhrsternes das Kunststoffbehältnis loslassen. Das so behaltene Kunststoffbehältnis wird anschließend durch Einschalten der Ladungsträgererzeugungsquelle bestrahlt.

Nach einer vorformlingsgrößenabhängigen Behandlungszeit wird der Bestrahlungsvorgang abgebrochen. Dies kann beispielsweise durch Ausschalten des Strahlstromes und vorteilhaft dem anschließenden Einleiten von Umgebungsluft erfolgen. Mit oder bzw. während des Druckausgleiches kann das Kunststoffbehältnis wiederum von einem Außengreifer eines Ausfuhrsternes erfasst werden. Dabei kann auch das Kunststoffbehältnis wieder nach unten abgezogen werden so dass die Ladungsträgererzeugungseinrichtung nicht beschädigt wird.

Eine eventuelle Außenentkeimung kann durch zusätzliche Strahlenemitter, wie beispielsweise einem Flächenstrahler, der der Innenbehandlung vor- oder nachgeschaltet ist, durchgeführt werden. Daneben wäre auch eine Außenbestrahlung der Kunststoffbehältnisse während der Innenentkeimung möglich.

Ein Aktivieren des Sterilisationssystems, d.h. der Ladungsträgererzeugungseinrichtung, kann dabei vor oder nach der Unterdruckerzeugung beginnen.

Fig. 3 zeigt eine Ausführungsform einer erfindungsgemäßen Vorrichtung. Auch hier ist wieder die Ladungsträgererzeugungseinrichtung 12 vorgesehen, welche zur Sterilisation der Behälterinnenseite dient. Das Bezugszeichen 16 bezieht sich auf die Halteeinrichtung, an der das Behältnis 10 durch Unterdruck angeordnet ist. Das Bezugszeichen 20 bezieht sich auf eine Druckbeaufschlagungseinrichtung, welche hier im Inneren des Kunststoffbehältnisses 10 ein Vakuum erzeugt, um dieses so an der Halteeinrichtung 16 zu befestigen. Ein großer Vorteil der erfindungsgemäßen Vorrichtung entsteht durch den hier vorgeschlagenen vergleichsweise aufwandlosen und schlanken Prozess. Die entstehende Störstrahlung ist minimal und auch der Energieaufwand ist gering. Das Bezugszeichen 10a bezieht sich auf eine Innenwandung des Kunststoffbehältnisses 10 und das Bezugszeichen 10b auf eine Öffnung bzw. Mündung des Kunststoffbehältnisses 10.

Das Bezugszeichen 32 kennzeichnet eine optionale Elektronenleiteinrichtung, welche die Elektronen in Richtung der Innenwandung der Kunststoffvorformlinge drängt. Diese Elektronenleiteinrichtung kann dabei als Hülse ausgebildet sein, welche mit positiver Ladung beaufschlagt wird. Auch wäre es möglich, dass die Elektronenleiteinrichtung, wie durch die gestrichelte Linie 34 angedeutet ist, nur in einem Bereich der Kunststoffvorformlinge angeordnet ist und die Kunststoffvorformlinge zusätzlich um ihre Längsrichtung L gedreht werden.

Es konnte festgestellt werden, dass eine ausreichende Sterilisation der Behältnisse nicht nur durch stark beschleunigte Ladungsträger möglich ist, sondern es bereits ausreichend sein kann, wenn die Ladungsträger auf die (Innen)-oberfläche der Kunststoffvorformlinge bzw. Behältnisse 10 gelangen. Das Bezugszeichen L kennzeichnet die Längsrichtung der Behältnisse 10.

Fig. 4 zeigt eine weitere Ausführungsform einer erfindungsgemäßen Vorrichtung. Neben dem elektrischen Leiter 22, der auch bei der Ausführungsform in Fig. 3 vorhanden ist, ist hier ein weiterer Leiter 24 vorgesehen, der hier als Ersatzemitter fungiert. Dieser Ersatzemitter kann dabei parallel oder versetzt zu dem Leiter 22 vorgesehen sein. Dabei kann, falls eine Steuereinrichtung eine Beschädigung des ersten Leiters 22 feststellt, der zweite Leiter 24 aktiviert werden, um auf diese Weise die Ladungsträger zu erzeugen.

Im Gegensatz zu den Vorrichtungen aus dem Stand der Technik entfallen hier eine aufwendige Elektronenbeschleunigungseinrichtung sowie auch eine Übergangsstrecke zwischen einem Strahlerausgang und dem Behältereingang. Somit besteht ein großer Unterschied darin, dass in der vorgestellten Erfindung eine Elektronenwolke mit nicht vorhandener oder nur minimaler Beschleunigungsspannung zur Sterilisation eingesetzt wird. Auf diese Weise ergeben sich mehrere Vorteile. Einerseits entfällt die bisherige Beschleunigungsspannung, wodurch eine Energieersparnis eintritt. Weiterhin wird die kinetische Energie der Wirkung in Elektronen reduziert und so eine Reduzierung der auftretenden Röntgenstrahlung erreicht. Daneben kann ein für die Erzeugung der Beschleunigungsspannung erforderlicher Hochspannungsgenerator entfallen, und auch die benötigte Abschirmdicke verringert werden und die Anlage kann in ihrer Gesamtheit vereinfacht werden, wobei auch eine Kostenreduzierung möglich ist.

Es wird damit bevorzugt in einem Feinvakuum eine Elektronenwolke erzeugt, die ohne oder mit einer deutlich reduzierten Beschleunigungsspannung zur umgebenden Behälteroberfläche transportiert wird.

### Bezugszeichenliste

- 1: Vorrichtung
- 2: Sterilisationseinrichtung
- 8: Träger
- 10: Behältnis
- 10a: Innenwand
- 10b: Öffnung des Behältnisses
- 12: Ladungsträgererzeugungseinrichtung
- 16: Halteeinrichtung
- 18: Welle
- 22: elektrischer Leiter
- 24: weiterer elektrischer Leiter
- 26: Hubeinrichtung
- 30a: Barrieren
- 32, 34: Elektronenleiteinrichtung

- L: Längsrichtung
- P1, P2: Pfeil

## Patentansprüche

1. Verfahren zum Sterilisieren von Behältnissen (10) und insbesondere von Kunststoffbehältnissen, wobei eine Innenwandung (10a) dieser Behältnisse (10) durch Beaufschlagung mit Ladungsträgern sterilisiert wird und wobei hierzu eine Sterilisationseinrichtung durch eine Öffnung (10b) des Behältnisses (10) in dieses Behältnis (10) eingeführt wird, und diese Sterilisationseinrichtung (2) eine Ladungsträgererzeugungseinrichtung (12) aufweist, wobei
die Ladungsträgererzeugungseinrichtung (12) wenigstens teilweise in das Behältnis (10) eingeführt wird und im Inneren des Behältnisses (10) die Ladungsträger erzeugt, wobei die Ladungsträger Elektronen sind, **dadurch gekennzeichnet, dass** innerhalb des Behältnisses (10) wenigstens zeitweise vor oder während des Sterilisationsvorgangs ein Unterdruck erzeugt wird, und die Ladungsträgererzeugungseinrichtung (12) einen ersten in das Behältnis (10) einführbaren stromdurchfließbaren elektrischen Leiter (22) umfasst.

2. Verfahren nach Anspruch 1,
**dadurch gekennzeichnet, dass**
im Inneren des Behältnisses (10) ein (Unter)Druck erzeugt wird, der zwischen 0,0001 mbar und 800mbar, bevorzugt zwischen 0,0003mbär und 600mbar, bevorzugt zwischen 0,001 mbar und 300mbar liegt.

3. Verfahren nach wenigstens einem der vorangegangenen Ansprüche,
**dadurch gekennzeichnet, dass**
das Behältnis (10) ein Kunststoffvorformling ist.

4. Verfahren nach wenigstens einem der vorangegangenen Ansprüche,
**dadurch gekennzeichnet, dass**
die Behältnisse während ihrer Sterilisation entlang eines vorgegebenen Transportpfads transportiert werden.

5. Verfahren nach wenigstens einem der vorangegangenen Ansprüche,
**dadurch gekennzeichnet, dass**
die Kunststoffbehältnisse (10) durch einen Unterdruck an einer Halteeinrichtung (16) gehalten werden.

6. Vorrichtung (1) zum Sterilisieren von Behältnissen (10) und insbesondere von Kunststoffbehältnissen (10), mit einer Sterilisationseinrichtung (2), welche eine Innenwandung (10a) der Kunststoffbehältnisse (10) mit Ladungsträgern beaufschlagt, wobei die Sterilisationseinrichtung (2) eine Ladungsträgererzeugungseinrichtung (12) aufweist, welche die Ladungsträger erzeugt, wobei die Ladungsträger Elektronen sind, wobei
die Ladungsträgererzeugungseinrichtung (12) derart gestaltet ist, dass sie in das Innere des Behältnisses (10) einführbar ist, wobei die Ladungsträgererzeugungseinrichtung (12) einen ersten in das Behältnis (10) einführbaren stromdurchfließbaren elektrischen Leiter (22) umfasst, **dadurch gekennzeichnet, dass** die Vorrichtung (1) eine Druckbeaufschlagungseinrichtung (20) aufweist, welche die Behältnisse (10) mit einem Unterdruck beaufschlagt.

7. Vorrichtung (1) nach Anspruch 6,
**dadurch gekennzeichnet, dass**
die Vorrichtung (1) eine Transporteinrichtung (8) aufweist, welche die Behältnisse (10) wenigstens zeitweise während deren Sterilisation transportiert.

8. Vorrichtung (1) nach wenigstens einem der vorangegangenen Ansprüche 6 - 7,
**dadurch gekennzeichnet, dass**
die Vorrichtung (1) eine Ablenkeinrichtung (32, 34) aufweist, welche die Ladungsträger in Richtung der Innenwand (10a) der Behältnisse (10) richtet.

9. Vorrichtung (1) nach wenigstens einem der vorangegangenen Ansprüche 6 - 8,
**dadurch gekennzeichnet, dass**
die Strahlungserzeugungseinrichtung (12) einen zweiten in das Behältnis (10) einführbaren stromdürchfließbaren elektrischen Leiter (24) aufweist.

## Claims

1. A method of sterilizing containers (10), and in particular of plastics material containers, wherein an inner wall (10a) of these containers (10) is sterilized by being acted upon with charge carriers, and wherein a sterilization device is introduced to this end into the container (10) through an opening (10b) in this container (10), and this sterilization device (2) has a charge carrier generation device (12), wherein the charge carrier generation device (12) is introduced into the container (10) at least in part and generates the charge carriers in the interior of the container (10), wherein the charge carriers are electrons, **characterized in that** an under-pressure is generated inside the container (10) at least for a time before or during the sterilization procedure and the charge carrier generation device (12) comprises a first electric conductor (22) which is able of being inserted in the container (10) and through which current can flow.

2. A method according to claim 1, **characterized in that** an (under-) pressure, which is between 0.0001 mbar and 800 mbar, preferably between 0.0003 mbar and 600 mbar, preferably between 0.001 mbar and 300 mbar, is generated in the interior of the container (10).

3. A method according to at least one of the preceding claims, **characterized in that** the container (10) is a plastics material pre-form.

4. A method according to at least one of the preceding claims, **characterized in that** the containers are conveyed along a pre-set conveying path during their sterilization.

5. A method according to at least one of the preceding claims, **characterized in that** the plastics material containers (10) are held on a holding device (16) by an under-pressure.

6. An apparatus (1) for the sterilization of containers (10), and in particular of plastics material containers (10), with a sterilization device (2) which acts upon an inner wall (10a) of the plastics material containers (10) with charge carriers, wherein the sterilization device (2) has a charge carrier generation device (12) which generates the charge carriers, wherein the charge carriers are electrons, wherein the charge carrier generation device (12) is designed in such a way that it is able of being inserted in the interior of the container (10), wherein the charge carrier generation device (12) comprises a first electric conductor (22) which is able of being inserted in the container (10) and through which current can flow, **characterized in that** the apparatus (1) comprises a pressure actuation device (20) which acts upon the containers (10) with an under-pressure.

7. An apparatus (1) according to claim 6, **characterized in that** the apparatus (1) has a conveying device (8) which conveys the containers (10) at least for a time during their sterilization.

8. An apparatus (1) according to at least one of the preceding claims 6 to 7, **characterized in that** the apparatus (1) has a deflecting device (32, 34) which directs the charge carriers in the direction of the inner wall (10a) of the containers (10).

9. An apparatus (1) according to at least one of the preceding claims 6 to 8, **characterized in that** the radiation generation device (12) has a second electrical conductor (24) which is capable of being introduced into the container (10) and through which current can flow.

## Revendications

1. Procédé de stérilisation de récipients (10) et en particulier de récipients en matière plastique, une paroi intérieure (10a) de ces récipients (10) étant stérilisée en étant soumise à l'action de porteurs de charge, et un dispositif de stérilisation étant introduit à cet effet dans ce récipient (10) à travers une ouverture (10b) du récipient (10), et ce dispositif de stérilisation (2) comprenant un dispositif de génération de porteurs de charge (12),
le dispositif de génération de porteurs de charge (12) étant introduit au moins en partie dans le récipient (10) et produisant les porteurs de charge à l'intérieur du récipient (10), les porteurs de charge étant des électrons, **caractérisé en ce qu'**un vide est créé à l'intérieur du récipient (10) au moins par intermittence avant ou pendant la procédure de stérilisation, et le dispositif de génération de porteurs de charge (12) comprend un premier conducteur électrique (22) pouvant être traversé par un courant et pouvant être introduit dans le récipient (10).

2. Procédé selon la revendication 1,
**caractérisé en ce qu'**
une pression (négative), laquelle est comprise entre 0,0001 mbar et 800 mbar, de préférence entre 0,0003 mbar et 600 mbar, de préférence entre 0,001 mbar et 300 mbar, est créée à l'intérieur du récipient (10).

3. Procédé selon au moins l'une des revendications précédentes,
**caractérisé en ce que**
le récipient (10) est une préforme en matière plastique.

4. Procédé selon au moins l'une des revendications précédentes,
**caractérisé en ce que**
les récipients sont transportés pendant leur stérilisation le long d'un trajet de transport prédéfini.

5. Procédé selon au moins l'une des revendications précédentes,
**caractérisé en ce que**
les récipients en matière plastique (10) sont retenus sur un dispositif de retenue (16) au moyen d'un vide.

6. Dispositif (1) de stérilisation de récipients (10) et en particulier de récipients en matière plastique (10), pourvu d'un dispositif de stérilisation (2), lequel soumet une paroi intérieure (10a) des récipients (10) en matière plastique à l'action de porteurs de charge, le dispositif de stérilisation (2) comprenant un dispositif de génération de porteurs de charge (12), lequel génère des porteurs de charge, les porteurs de charge étant des électrons,
le dispositif de génération de porteurs de charge (12) étant conçu de telle manière qu'il peut être introduit à l'intérieur du récipient (10), le dispositif de génération de porteurs de charge (12) comprenant un premier conducteur électrique (22) pouvant être traversé par un courant et pouvant être introduit dans le récipient (10), **caractérisé en ce que** le dispositif (1) comprend un système de soumission en pression (20), lequel soumet les récipients (10) à l'action d'un vide.

7. Dispositif (1) selon la revendication 6,
**caractérisé en ce que**
le dispositif (1) comprend un système de transport (8), lequel transporte les récipients (10) au moins par intermittence pendant leur stérilisation.

8. Dispositif (1) selon au moins l'une des revendications précédentes 6 à 7,
**caractérisé en ce que**
le dispositif (1) comprend un système de déviation (32, 34), lequel oriente les porteurs de charge en direction de la paroi intérieure (10a) des récipients (10).

9. Dispositif (1) selon au moins l'une des revendications précédentes 6 à 8,
**caractérisé en ce que**
le dispositif de génération de rayonnement (12) comprend un deuxième conducteur électrique (24) pouvant être traversé par un courant et pouvant être introduit dans le récipient (10).
